(19)

**Europäisches Patentamt**
**European Patent Office**
**Office européen des brevets**

(11) **EP 4 527 363 A1**

(12) **EUROPEAN PATENT APPLICATION**

(43) Date of publication:
26.03.2025 Bulletin 2025/13

(51) International Patent Classification (IPC):
**A61F 9/008** (2006.01)

(21) Application number: 23198985.6

(22) Date of filing: **22.09.2023**

(52) Cooperative Patent Classification (CPC):
**A61F 9/008;** A61F 9/00821; A61F 2009/00863

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **OD-OS MacuTherm GmbH**
**14513 Teltow (DE)**

(72) Inventors:
• **Rolfs, Janine**
  **15711 Königs Wusterhausen (DE)**
• **Rose, Arnd**
  **14532 Stahnsdorf (DE)**
• **Teiwes, Winfried**
  **14532 Kleinmachnow (DE)**

(74) Representative: **Pfenning, Meinig & Partner mbB**
**Patent- und Rechtsanwälte**
**Joachimsthaler Straße 10-12**
**10719 Berlin (DE)**

(54) **DEVICE AND METHOD FOR LASER TREATMENT OF THE RETINA OF AN EYE WITH A LASER POWER ABOVE THE EYE SAFETY THRESHOLD**

(57) The invention relates to a device and method for tissue treatment of the retina (16) of a patient's eye (4) by means of a controllable laser (2), the laser beam (11) of which can be directed onto different treatment area regions (20a, 20b, 20c) of the retina, characterized in that the device is set up to heat the retina spot (26a, 26b) by spot, within one or more treatment area regions, successively by irradiation with the laser for a controllable period of time to a temperature between 50 and 55 degrees Celsius.

Fig.1

EP 4 527 363 A1

**Description**

The invention is in the field of medical technology, in particular ophthalmology, and can be used with particular advantages in laser eye treatments, for example in the treatment of age-related macular degeneration.

Eye treatments by means of laser are meanwhile common for a multiplicity of illnesses and for the pursuit of different goals. In particular, treatments of the retina by a laser are well-known. Often such treatment is aimed at tissue alteration. The effect is largely based on the controlled input of energy in the form of light energy into the retina or tissue volumes close to the retina in order to achieve coagulation of cells or other tissue changes there, for example by increasing the temperature. In some applications, the laser power can be selected so high that cells are destroyed thermally (rupture treatment).

Laser treatments of the eye of a patient must be planned diligently due to the existing risks and the complex tasks in order to prevent or minimize damage to tissue regions that shall not be treated. For this reason, such treatments must be carried out as far as possible by physicians or under the close supervision of physicians. In particular, it is a paradigm that the laser power of the treatment laser must be well controlled and adjusted to the individual conditions of the patient in order to achieve specific tissue changes while avoiding unwanted tissue damage. For this purpose, various control and examination methods have been developed to determine the physiological conditions in the patient's eye prior to treatment.

Often, the individual treatment conditions in the patient's eye are tested by so-called titration shots with the treatment laser, whereby a visible tissue change is monitored after the titration shots. The visible results are then used to calibrate the treatment laser.

In some cases, prior to retinal treatment, an image of the retina of the eye potentially to be treated is first taken or recorded and a decision is made as to the need for and/or type of treatment based on conditions discernible by means of the image.

From the European patent application EP 1875 857 A1 an ophthalmoscope with imaging optics and an illumination device as well as an optical sensor is known, with which images of the retina of an eye can be recorded. Such an ophthalmoscope can be used, for example, for planning a laser treatment by a physician.

Against the background of the state of the art, the present invention aims at simplifying the planning and processing of retinal treatments.

This task is solved with the features of the invention according to the independent patent claims. The dependent patent claims relate to specific embodiments of the invention.

Accordingly, the invention relates to a device for tissue treatment of the retina of a patient's eye by means of a controllable light source, in particular a laser, the light beam of which can be directed onto different treatment area regions of the retina, wherein the device is set up to heat the retina spot by spot, within one or more treatment area regions, successively by irradiation with the light source for a controllable period of time to a temperature between 48 and 58 degrees Celsius, in particular between 50 and 55 degrees Celsius.

According to medical treatment guidelines, early-stage retinal diseases should not be treated by laser, as this destroys or at least damages tissue in the known forms of therapy.

However, even if the temperatures reached by the treatment by the device according to the invention are usually above the eye safety threshold of laser operation, they remain below temperatures at which immediate or delayed tissue alteration is caused by the laser. At the same time, said heating according to the invention initiates or accelerates physiological processes in the tissue that have a positive effect on the health and functioning of the target tissue. A temperature of between 48 and 58 degrees Celsius, in particular between 50 and 55 degrees shall be kept for each single laser spot for a controlled time and not be exceeded at all. Thereby, for example, enzymes, hormones and neurotransmitters are released or their function is enhanced, which improve tissue functionality and thus prevent or slow down the progression of retinal diseases. This can be achieved without the need for intravital injection of drugs or other costly intervention.

The achievement of the desired temperatures of the retina can be adjusted either by empirical values of the irradiation parameters of the light source, which may be a laser or a light emitting diode (LED), which can also be adjusted, for example, by a self-learning system. However, a non-invasive real-time measurement and monitoring of the temperature at the respective irradiation site can also be provided by a spectroscopic method. According to the described method, a reaction of the tissue to a short radiation pulse is observed in the form of transient pressure signals of a pressure wave, whereby the signals allow a temperature determination of the tissue via their dependence on the so-called Grüneisen coefficient.

A heating up spot by spot may in some cases mean that a light beam jumps from spot to spot, remains for a certain time without movement and heats every spot up, but it may as well include a continuous or nearly continuous movement of the light beam with a continuously moving spot on the tissue.

The invention also relates to a device for tissue treatment of the retina of a patient's eye by means of a controllable light source, in particular a laser, the light beam of which can be directed onto different treatment area regions of the retina, characterized in that the device is set up to heat up one or more treatment area regions in a manner that does not lead to a damaging or coagulation of tissue of the retina but leads to the release of a cascade of biochemical reactions, in particular leading to an improved metabolism of the RPE, further in particular leading to the release of heat shock proteins in or near the retina.

Such a device is intended to stimulate and heat the RPE (retinal pigment epithelium) in a targeted manner with an energy input by irradiation without damaging the overlying photoreceptors. The aim is to trigger a mechanism of action that results in improved metabolism of the RPE and thus improved degradation of disease-causing deposits such as drusen. An automatable treatment schedule makes this treatment easy to perform, counteracting a common cause of blindness in old age.

Controlled and selective stimulation of the RPE by the device according to the invention improves the metabolism of the RPE cells themselves, leading in many cases to improved antioxidant function and increased cell protection through an increase in heat shock proteins. In addition, autophagy function in RPE cells can also be improved by such "hormetic" thermal stimulation. Lysosomes, intracellular organelles, which digest unwanted substances in cells, are essential for autophagy. Improved lysosomal function caused by the irradiation according to the invention also leads to less new lipid deposition inside and outside RPE cells. Improved lysosomal function also leads to improved function of photoreceptor cells, for whose constant renewal the RPE is responsible. In addition, RPE cells release anti-inflammatory substances, angiogenesis inhibitors and other substances, in the surrounding tissues.

This results in a thinning of Bruch's membrane below the RPE. This increases its permeability and thus improves the supply of the RPE and the photoreceptors above it. The removal of metabolic waste is improved, which in turn leads to a reduction in the occurrence of drusen in the retina.

In one embodiment of the invention, it may be provided that the device is arranged to heat the retina spot by spot successively by irradiation with the light source for a period of less than 500 msec, in particular less than 200 msec per spot, to a temperature between 48 and 58 degrees Celsius, in particular between 50 and 55 degrees Celsius.

It may further be provided that the period of irradiation with the said power is at least 10 msec, further in particular at least 50 msec, further in particular at least 100 or 150 msec.

For example, time periods between 50 and 200 msec or between 50 and 500 msec or between 100 and 200 msec or between 100 and 500 msec or between 150 and 500 msec may thus result.

By maintaining an optimum time window, it can be ensured on the one hand that a target temperature is reached in the temperature window and on the other hand that critical limit temperatures are not exceeded, so that tissue damage can be reliably avoided.

The time windows described above may in some cases mean that each point on the retina is exposed to the light beam of the light source for the indicated time period no matter if the light beam is moving continuously or step by step.

The task can also be solved by a device for tissue treatment of the retina of a patient's eye by means of a controllable light source, in particular a laser, the light beam of which can be directed onto different treatment area regions of the retina, characterized in that the device is set up to irradiate the retina spot by spot, within one or more treatment area regions, successively by irradiation with the light source for a period of less than 500 msec, in particular less than 200 msec per spot, with a power density of between 100 and 350 W/cm$^2$, in particular between 120 and 300 W/cm$^2$.

Also in this case, the above mentioned lower limits for the irradiation time with the mentioned power density can advantageously apply as well as the time windows mentioned further above.

The specified power densities of the light source, in particular the laser or LED cause the tissue to reach a temperature for a suitable period of time, at which physiological processes in the tissue are initiated or accelerated by the heating, which have a positive effect on the health and functioning of the target tissue without damaging the tissue. In this process, enzymes, hormones and messenger substances are generated or increased, which improve the tissue functionality and thus prevent or slow down the progress of retinal diseases.

In a further embodiment, it can be provided that the device is arranged to assign the treatment area regions to be treated with the light source to predefined area sections and to irradiate them according to a sequence determined by the assignment of the treatment area regions to the area sections.

It may also be provided that at least one group of area sections are arranged next to one another in the circumferential direction around the fovea and/or in that the area sections together cover an area of the retina surrounding the fovea, the extent of which is in particular such that it does not cover the exit site of the optic nerve and in particular the arcades.

In some cases, it will be useful to treat the whole retina of a patient according to the conditions described above. Therein, some regions may be excluded which are sensitive to laser treatment or where a laser treatment is not useful, like for example the optical nerve head, the fovea or areas of the retina where big arteries are located. In other cases, it may be useful to select certain areas of the retina for treatment.

In these cases, in order to enable optimized planning and control of the treatment laser for retinal treatment in the sense of optimized logistics, area sections are predefined to which the treatment area regions are assigned. This makes it easier, more reliable and easier reproducible to identify and locate the treatment area regions on the retina in which the retina is to be treated. The treatment laser can also be controlled more easily with reference to the predefined area sections. The treatment area regions can be freely positioned on the retina, but they can be easily and quickly located and controlled by the treatment laser or another light source like an LED through their assignment to predefined area sections. A further advantage of the use of predefined area sections is that they permit a standardization for location information on the retina, so that, for example, treatment area regions can be defined by means of a first system and the position of the treatment area regions can be found and controlled again by means of another system and/or with a time shift. An already known system of such predefined area sections is given by the so-called ETDRS-grid, which is already used in the area of the macula to categorize diagnostic information, the results of functional tests and for example also the results of layer thickness measurements by optical coherence tomography.

Advantageously, the device can be set up in such a way that the laser sequentially irradiates the predefined area sections in a predetermined order or according to a predetermined rule.

By assigning a treatment according to the invention to the predefined area sections or by assigning selected treatment area regions to the predefined area sections, the treatment can be easily processed and treatment area regions can be easily targeted by the treatment laser. A sequence of irradiations can also be defined in advance of the treatment or already during programming of the device according to the assignment to the area sections, so that the changes in setting, for example beam angle changes, of the laser from one area section to another area section and thus also from one treatment area region to the next or generally movements of the laser beam can be minimized. This minimizes both irradiation inaccuracies and the time required for the overall treatment.

It can also be provided that the predefined area sections jointly cover an area surrounding the fovea, excluding the fovea, the extent of which is such that it does not cover the exit site of the optical nerve and in particular the arcades.

The predefined area sections can surround the fovea concentrically, so that the fovea forms the center of the entire surface covered by the area sections. In this context, the arcades are to be understood as the large blood vessels that arcuately surround the field of vision on the retina and emanate from the point of exit of the optic nerve into the retina.

A further embodiment can, for example, provide that the light source control is set up to irradiate a group of area sections, in particular all area sections individually one after the other, either not at all or to cover 100% of the individual area section.

Since the laser treatment according to the rules described above cannot cause any damage in the tissue of the retina, the irradiation of additional tissue is also harmless, even if it extends the treatment time. Therefore, as long as laser treatment is useful in a treatment area region within an area section, the entire area section can also be irradiated, i.e. covered by laser spots to be irradiated. This makes treatment planning particularly simple, since the boundaries of the area sections are fixed and patterns for covering entire area sections with laser spots/irradiation spots can also be stored in advance.

In a further embodiment, it can also be provided that at least one group of area sections are arranged next to each other in the circumferential direction around the fovea.

In such an arrangement, the individual area sections or the treatment area regions arranged on them can be approached and processed successively with minimized effort by the treatment laser/treatment laser beam during the laser treatment. For example, directly adjacent area sections or the treatment area regions arranged on adjacent area sections can be treated directly one after the other. The fovea itself is excluded, since it is not included in the area sections and is not covered by them.

A further useful embodiment of the invention can provide that the area sections are distributed around the fovea, in particular in the form of segments of at least one circular ring concentrically surrounding the fovea, or of an elliptical ring surrounding the fovea, or in that the area sections are arranged on one or more segments of a ring, in particular circular ring, concentrically surrounding the fovea, wherein the area sections are arranged rotationally symmetrically with an n-numbered rotational symmetry, wherein n is in particular between 1 and 9 and is further in particular 2, 4 or 6.

A rotationally symmetrical arrangement of the area sections with n-count symmetry results in a clear arrangement and addressability of the area sections. For example, they can be distributed around the fovea in a regular polygon, in particular a quadrilateral, pentagon or hexagon.

It can also be advantageously provided that the device is set up to record areas of the retina with an imaging method, to select treatment area regions on the basis of an image of the retina, to assign irradiation parameters to these and to irradiate these using the irradiation parameters.

For this purpose, the device according to the invention can have a retina recording device which records an image of the predefined area sections of the retina by means of an imaging process, in particular by means of a camera or an optical scanner, and assigns irradiation parameters associated with the respective area sections.

With such an image, the pigmentation strength of the retina can basically be determined, which also determines the absorption strength of a laser beam on the retina, so that on the basis of a determined pigmentation strength, the laser intensity, in particular the laser power density, or a duty cycle of the laser modulation can be set in such a way that a desired power density is achieved and kept in the area of the retina at a respective laser spot. These laser parameters can also be determined and applied uniformly for each area section.

Irregularities of the retina can also be detected based on the image data, which can lead to an increased need for treatment or to a location-dependent laser absorption strength or to the identification of areas which shall not be treated. Based on the detected location-dependent data, the laser intensity or power can also be controlled based on location. Alternatively, a patient's retina can be imaged prior to treatment and also in a different device and the acquired data can be transmitted to the device set up for treatment.

The irradiation parameters may be within the limits indicated above, and the retinal capture/image data may be used to amend or correct the irradiation parameters within the limits indicated.

In the device according to the invention, the invention may further provide an eye tracking device which compensates for movements of the eye during irradiation of the treatment areas and/or a temperature measurement device may be provided comprising a thermo-optic spectrometer which sends laser pulses to the retina and registers pressure waves being emitted by the retina as a response signal.

Once the areas of the retina to be treated are determined and the light source/laser/treatment laser is programmed, any eye movement may cause the retina to move laterally under the laser beam, thereby exposing to the laser beam areas of the retina that are not worthy of treatment or areas that should not be irradiated with the laser.

For this reason, so-called eye tracking is provided, i.e. the movements of the treated eye are captured and the steering of the beam of the treatment laser is adjusted in such a way that the target areas of the retina are reliably hit despite the eye movement. The eye movement can be detected, for example, either by means of a camera or by the detection of reflected scanning beams.

The temperature measurement device is based on the "Grüneisen" formula and measures the absorption of laser radiation pulses by the response signal in the form of pressure waves, which are created by the sudden temperature rise or fall. The temperature measurement device can be used to establish a closed loop with the laser control and reliably limit the temperature at a current laser spot to the target temperature window.

In addition to a device of the type described above, the invention also relates to a method for tissue treatment of the retina of a patient's eye by means of a controllable light source, in particular a laser, wherein the light beam can be directed onto different treatment area regions of the retina, wherein within one or more treatment area regions the retina is heated spot by spot one after the other by irradiation with the light source for a controllable period of time to a temperature between 48 and 58, in particular between 50 and 55 degrees Celsius

A further implementation of the method may provide that the treatment area regions are heated up in a manner that does not lead to a damaging or coagulation of tissue of the retina but leads to the release of a cascade of biochemical reactions, in particular leading to an improved metabolism of the RPE, further in particular leading to the release of heat shock proteins in or near the retina.

In addition, the invention relates to a method for tissue treatment of the retina of an eye of a patient by means of a controllable light source, in particular a laser, wherein the light beam can be directed onto different treatment area regions of the retina, characterized in that within one or more treatment area regions the retina is irradiated spot by spot in succession by irradiation with the light source for a period of less than 500 msec, in particular less than 200 msec per spot, with a power density between 100 and 350 $W/cm^2$, in particular between 120 and 300 $W/cm^2$.

Also in this case it can be further provided that the period of irradiation with said power is at least 10 msec, further in particular at least 50 msec, further in particular at least 100 or 150 msec.

For example, time periods between 50 and 200 msec or between 50 and 500 msec, between 100 and 200 msec, between 100 and 500 msec, or between 150 and 500 msec can thus result.

By maintaining an optimum time window, the process can also ensure, on the one hand, that a target temperature is reached in the temperature window and, on the other hand, that critical limit temperatures are not exceeded, so that tissue damage can be reliably avoided.

The selected treatment area regions can cover, e.g. the whole retina, in some cases with the exception of some of the areas for which a treatment is not necessarily useful, like for example the fovea, the optical nerve head and the surface of the larger blood vessels in the retina.

A particular embodiment can also provide, in the above-mentioned methods, that the treatment area regions to be treated with the light source are assigned to predefined area sections and are irradiated according to a sequence determined by the assignment of the treatment area regions to the area sections.

In a further embodiment of the invention, it can also be provided that first an image of the predefined area sections of the retina is recorded by means of a retinal recording or scanning device by means of an imaging method and irradiation parameters associated with the entire retina or with the area sections or treatment area regions determined within these are assigned.

More concretely, it can also be provided that the retina is illuminated completely or in part with an illumination device under defined illumination parameters and during and/or shortly after the illumination at least one optical image, in particular at least one camera image, of at least one target area of the retina is recorded and in which optimized irradiation parameters for the irradiation of one or more target areas, for example treatment area regions, of the retina are determined using the image(s) or camera images.

The method and a device set up for carrying out the method in the way described above are intended to make it possible to determine irradiation parameters for operating a light source, in particular a laser, as quickly as possible, in as automated and reproducible a manner as possible. This can be achieved immediately before the treatment by the laser irradiation within the described device, but also at a greater time distance before the treatment, in some cases also at a different location than the laser or light source treatment takes place. The determined irradiation parameters or their correction values or physiological measured values, which serve to determine these quantities, can be stored and made available for retrieval by a device for laser irradiation according to the invention, for example in an accessible computer network or a communication network.

This also applies to the correction values described further below, which can be determined by various types of measurements and measuring devices.

According to the prior art, it is common practice to experimentally apply laser shots (titration shots) to areas of the retina to determine the tissue response in the individual patient. Often, the surgeon then assesses the degree of tissue discoloration due to the laser effect according to his personal impression and, based on this, adjusts the laser parameter for irradiation during the actual treatment.

With the method described here, it is possible to completely dispense with a trial operation of the laser before the treatment. In some cases, by additionally using images or camera images of the retina obtained in real time, an assessment of the individual retinal tissue is further objectified, transparent, traceable, reproducible and verifiable.

Thus, the laser power density $j$ or light energy density necessary or useful for the irradiation can further be adjusted or corrected for the individual treatment for the whole or a part of the retina or also depending on the location of the irradiation on the retina.

The illumination device can be a source of visible light, for example a light emitting diode or a laser, for example also the treatment laser itself, a surface illumination, for example by means of an illuminated reflecting surface, a frosted glass pane illuminated from the rear or a light diode matrix or another broadband light source. Illumination beyond the visible range of the optical spectrum in the infrared or ultraviolet range can also be considered. In this context, the defined illumination parameters can be understood as, for example, a light intensity and the wavelength or a wavelength distribution of the emitted radiation. These can be set to specific values or characteristics, for example. The illumination parameters, such as the illumination intensity, distribution of the illumination intensity, and the spectrum/wavelength distribution, can alternatively or additionally also be measured, and the measured values can then be used in the evaluation of the recordings. In addition, it should be noted that radiation in the non-visible range, such as in the infrared range, can also be used, whereby an image captured in this process or a camera image can also be an infrared image that is captured with corresponding detectors. For example, hyperspectral sensors can also be used for the recordings. For the illumination, certain areas of the retina, for example treatment area regions, can be selected automatically or manually. In the case of automatic selection, certain geometric illumination patterns can be predefined and selectable. The method can also use raster-detecting/scanning methods of illumination and scanning, in which, for example, the target areas/area sections or the entire retina are illuminated with a focused light source, each covering only a narrowly limited area, and the reflection is detected by means of a light-sensitive, non-location-resolving sensor, for example a photodiode. In this way, the entire area of the retina can be continuously optically scanned and an optical image of the reflectivity and/or absorption strength can be created. Conversely, the entire retina can also be illuminated simultaneously and, by means of a spatially resolving optical sensor, the light reflected in each case from a limited surface area of the retina can be continuously/scanningly recorded in all areas of the retina or in the potential treatment area regions/target areas, and in this way an optical image of the reflectivity and/or absorption strength of the retina can be created.

In the context of the present invention, an optical recording can also be understood, for example, as an optical image of the retina.

Imaging methods for capturing an image or camera image of the retina may also include, for example, the methods of fundus photogravure, fundus reflectometry, autofluorescence imaging, photoacoustic imaging, and functional optical coherence tomography with corresponding illumination devices and illumination methods. These methods can be used, for example, to determine the pigmentation strength of the retina, which is decisive for the absorption strength of the subsequently used treatment beam or to detect areas of the retina that should not be treated. However, the pigmentation strength can also be determined in general from the brightness of the retina, which is captured by the image or the camera image.

Often, only certain treatment area regions/target areas of the retina are to be treated with a laser beam. In this case, it may be sufficient to illuminate the target areas/treatment area regions and capture an image or a camera image of these target areas. However, it may also be useful to capture images/camera images of the entire retina, since the necessary irradiance of the laser or other light source during treatment can be estimated taking into account the pigmentation thickness even in other areas of the retina not intended for treatment. Other, additional non-local characteristics of the person to be treated can also be taken into account when determining the necessary irradiance. Further information on this topic will be provided below.

For the optimization of a device or method of the type described above, it may also be provided that, for the determination of the irradiation parameters, the intensity of the pigmentation for one or more target areas of the retina and/or the intensity distribution of the pigmentation on the retina are determined by means of the image(s) or the camera image(s).

It can also be provided that, in order to determine the irradiation parameters for one or more treatment area regions of the retina, additional correction values are determined with respect to the expected absorption and/or scattering of the treatment beam in the vitreous body of the eye on the way to the target regions/treatment area regions to be irradiated, the correction values being determined in particular from the optical quality of the optical image, further in particular from an image sharpness or an image contrast.

The sharpness or contrast of the image can be assessed based on imaged inhomogeneities of the retina, such as blood vessels or nerve nodes. The sharpness/contrast information obtained from different areas of the retina can be compared with each other and/or with stored reference images in order to assess the influence of scattering/absorption in the vitreous body of the eye on the path to the retinal area in question and to determine this parameter generally for an eye or also as a function of the location on the retina.

For example, it can be advantageous for the optimization of a device or a method of the type described above to detect the melanin pigmentation of the retina and for this purpose to irradiate the retina with light in the visible range with wavelengths or even exclusively with wavelengths which are particularly well absorbed by the melanin pigmentation.

Wavelengths of illumination and/or detection in the range between 450 nm and 1064 nm can be advantageous for a measurement. In particular, measurements at 517nm, 532nm or 577nm can be advantageous. The scattering and absorption of radiation by melanin is strong in this range. In addition, the wavelengths at which the differences between the absorption strengths of melanin and blood are significant may also be advantageous. It may be useful to compare images taken at radiation with a wavelength of 480 nm (local minimum of absorption in blood) with images taken at 550 nm (local maximum of absorption in blood). As reference values in reflectance measurements, for example, the papilla can be used as a nerve node that shows a strong reflection in a healthy state or areas with blood vessels in which the presence of oxygen-rich or oxygen-poor blood can be selectively detected and useful comparison values can be generated when using suitable wavelengths that can detect these blood variants.

During laser treatment of the retina, the treatment beam is partially absorbed or scattered due to the non-hundred percent transparency of the vitreous body of the eye. This effect can be taken into account when determining the irradiation parameters for the laser, in such a way that the laser intensity is selected somewhat higher than is directly necessary for the treatment of the retina, if necessary to compensate for this. The absorption of the treatment beam depends on the individual and local characteristics of the vitreous body of the patient to be treated. In the context of the invention, the absorption can be estimated because the illuminance with which the retina is illuminated is known and the backscattered signal, for example the reflected light, depends in terms of its intensity both on the pigmentation strength of the retina and on the absorption of the light in the vitreous body. In order to be able to separate the two effects influencing the backscattering from each other, several measurements can be performed under changed optical conditions, like, for example, irradiating the retina by a light beam under different angles or by light beams with different focus spot diameters.

In many cases it can be useful that for the generation of a camera image the retina is illuminated with a radiation of known wavelength distribution, which comprises in particular an infrared radiation and/or light in a wavelength range absorbed by melanin, wherein additionally the light intensity of the illumination device and/or the total light power impinging on a unit area of the retina and/or the light power impinging on a unit area of the retina is determined in a defined wavelength range and wherein the brightness values and/or colours of the camera image are used to determine the parameters for the irradiation of the retina.

From empirical values for the necessary intensity of the treatment beam in connection with previously measured brightness values of the retina, irradiation parameters can be determined for the individual case, whereby, for example, the absorption of the treatment beam by the vitreous body can be represented by an assumed constant quantity or factor which, for example, can depend, among other things, on the age of the patient.

In addition, it can be advantageously provided that for one or more target areas the thickness of the retina is determined and taken into account in the determination of the irradiation parameters, the determination of the thickness of the retina being carried out in particular by the creation of an OCT thickness map of the retina, and/or that for the determination of the irradiation parameters the type and intensity of the pigmentation of body regions of the person to be treated which differ from the retina, in particular the type and intensity of the pigmentation of the iris of the eye or of the skin or of the hair of the individual patient, are used.

By means of an OCT (optical coherence tomography) measurement, the thickness of the retina can be determined in a location-dependent manner, for example to detect oedema in the retina, which often itself absorb part of the laser radiation, so that during the actual treatment certain portions of the treatment beam do not reach the pigment layer. Thus, the OCT thickness map for the irradiation parameters can be used to determine a location-dependent correction value for a laser power over the total area or partial areas of the retina.

In another implementation for the optimization of an apparatus or method of the type described above, it can also be provided that, in addition to the captured image(s) or camera images, one or more predetermined or predeterminable reference images or reference images are used to determine the irradiation parameters.

The reference images can, for example, represent target states of the retina which can be compared with the captured images/camera images in order to perform a difference analysis and to determine a necessary irradiation intensity or useful irradiation parameters from the differences between a captured image and a reference image. The irradiation parameters that determine the treatment intensity or irradiation intensity can typically include the strength of the laser beam or other light source, i.e. its power or power density, and/ or the size of the laser beam or light beam spot, as well as the time duration and number or duty cycle of emitted laser pulses and pauses between the laser pulses. Finally, reference images can also be taken, for example, from databases or obtained from previously acquired and stored images/camera images of the same patient.

In a further implementation for the optimization of a device or a method of the type described above, it can also be envisaged that, in order to determine the irradiation parameters for the whole retina or for one or more area sections or treatment area regions of the retina to be irradiated, on the one hand the intensity of the pigmentation of the target area(s) and on the other hand the expected absorption and scattering of the light forming the treatment beam in the eye lens on the way to the target areas are determined independently of one another by carrying out at least two measurements under different illumination conditions, wherein in the two or more measurements in particular at least one of the following parameters is varied: Size of the focused light spot on the retina, wherein in each case the deviation from an expected light intensity distribution on the area of the focus spot is measured, direction of incidence of an illumination beam of the illumination device through the pupil and the vitreous body for illumination of the respective target area, direction of incidence to the target area and/or direction of exit of a detected reflected illumination beam of the illumination device, wavelength range or wavelength distribution of an illumination beam of the illumination device.

This method allows the determination of the distribution of the pigmentation thickness on the retina on the one hand and the determination of the absorption and scattering of a laser beam or light beam by the vitreous body as a function of the laser target point on the retina and the light path on the other hand independently of each other. For such a measurement, for example, the illumination light path can be changed in several successive individual measurements when illuminating a target area of the retina, but the same target area can be illuminated and recorded in each case. On the other hand, a target area of the retina can also be illuminated in different ways by the illumination device in several measurements with a constant light path, so that the influence of the reflectivity of the retina can be varied by illumination with different spectra or by light in different wavelength ranges.

Alternatively or additionally, it can also be provided that, in order to determine the irradiation parameters for one or more treatment area regions/target areas of the retina to be irradiated, on the one hand the intensity of the pigmentation of the target area(s) and/or the expected absorption and/or scattering of the treatment beam in the eye lens on the way to the target areas are determined, by performing at least two measurements, one of the measurements being directed to the absorption and/or reflection characteristics in the area of the optical nerve head and/or at least one blood vessel or part of a blood vessel in the retina, while a second measurement is directed to the absorption and/or reflection characteristics in one of the remaining areas of the retina to be irradiated.

In order to optimize a device or a method of the type described above, the invention can also provide that, in order to determine the irradiation parameters for one or more treatment area regions/target areas of the retina to be irradiated, at least two locations of the retina are in each case illuminated successively or simultaneously with different illumination characteristics, in particular different illumination intensities, and in each case the intensity of the reflected signal is recorded, wherein, for the locations measured in this way, in each case the functional relationship between the illumination characteristic, in particular the illumination intensity, and the intensity of the reflected signal is determined, in particular the illuminance and the intensity of the reflected signal, in particular in the case of a linear relationship the slope of the functional curve, is determined for the locations measured in this way, and wherein the ratio of the reflectivities at the locations measured is determined by comparison of the functional relationships at the locations measured, in particular the slopes of the functional curves, wherein in particular one of the locations lies on the surface of the optical nerve head or a blood vessel or at least partially covers it.

The optic nerve head (optic disc) is usually not pigmented and reflects light much more strongly than the pigmented parts of the retina. In addition, the reflectivity in the optical nerve head is only slightly dependent on characteristics of the retina of an individual patient. Thus, the optical nerve head can serve as a "reflectance standard" or reference for a comparative measurement of the intensity of pigmentation.

The blood vessels of the retina can also be detected and used for a comparative measurement in the areas of the retina defined by them. The guided blood has a certain different absorption characteristic than melanin.

Absorption also depends on whether the blood is saturated with oxygen or not, that is, what type of vessels are irradiated. In order to be able to measure and evaluate these properties of absorption and reflectivity in the blood vessels in a differentiated manner, it can be useful to perform a reflectivity or absorption measurement at wavelengths of the illumination light that are particularly strongly absorbed by the oxygen-saturated or the oxygen-deficient blood. Alternatively to the corresponding selection of the illumination light, the corresponding interesting wavelengths can also be selected on the detection side.

It has been found that the functional relationship between an illumination characteristic, in particular the illumination intensity on the one hand, and the intensity of the reflected radiation on the other hand, at a location of the retina allows a good determination of a value representing the reflectivity of the retina at this location. In the case of a linear correlation between the illuminance and the intensity of the reflected radiation, for example, the linear regression method can be used to determine a slope of the straight line forming the function graph for a plurality of measurements in order to compensate for measurement errors. The slopes of the straight lines, which can then be assigned to the respective retinal locations measured, can form a relative measure of reflectivity. By comparison with a corresponding relation, in particular a straight line slope, at a location where the reflectivity is known or can be well estimated, the reflectivity values assigned to the measurement locations can then be calibrated. The reflectivity at a retinal location can then be used to determine the absorption strength for a treatment beam.

The wavelength of the radiation illuminating the respective measurement location can also serve as a variable illumination characteristic, so that in each case two locations are successively illuminated with radiation of different wavelengths, the intensity of the reflected radiation is measured in each case, and the functional relationship is determined and compared for the two locations.

The differentiation of the reflectivity at different wavelengths can be done both by illumination successively with different wavelengths with simultaneous broadband detection (or detection adapted by a wavelength window) and with broadband illumination covering a continuous spectrum or with illumination by a laser array comprising a plurality of individual wavelengths. In the latter case of broadband excitation/irradiation, wavelength-selective detection of the reflected radiation shall be provided, for example by defining colour channels, using wavelength-selective filters or using a spectrometer or a multispectral camera.

Since the reflectivity in the area of the optical nerve head varies only slightly, a deviation of the determined characteristic and the reflectivity value determined in a first step from a reference value can be attributed to the influence of the absorption and/or the scattering of the radiation on the measurement path through the eye of the individual patient.

Deviations of the measured values from the reference value of the reflectivity at a reference location such as the optic disc or the blood vessels can thus be used to determine the attenuation due to absorption and/or scattering in the eye body.

For example, it can also be provided that when determining the irradiation parameters or correction factors for all treatment area regions, a calibration is performed with one or more of the following calibration parameters of the respective person to be treated: measured integral pigmentation strength of the skin, measured integral pigmentation strength of the iris of the eye, measured pigmentation strength of the hair (main hair, eyebrows), colour of the skin pigmentation, colour of the iris, colour of the hair of the individual patient. Such a calibration can also provide, for example, that the listed quantities are not included in the algorithm for determining the irradiation parameters, but that after determining an irradiation intensity, a calibration of the results with the mentioned quantities is carried out, for example by multiplying the irradiation intensity, given by the laser energy and the length and duty cycle of the laser pulse, by a certain calibration factor.

In the following, the invention is shown by means of figures of a drawing and further described below.

Therein shows

| | |
|---|---|
| Figure 1: | a device for tissue treatment of the retina with a laser according to the invention, |
| Figure 2: | a laser treatment device with a temperature control, |
| Figure 3: | a diagram about useful power density ranges for retina laser treatment, |
| Figure 4, 5, 6: | area sections of the retina that can be predefined, |
| Figure 7: | area sections with a treatment area region and laser spots located in the treatment area region, and |
| Figure 8: | a retina laser treatment device with an imaging device. |

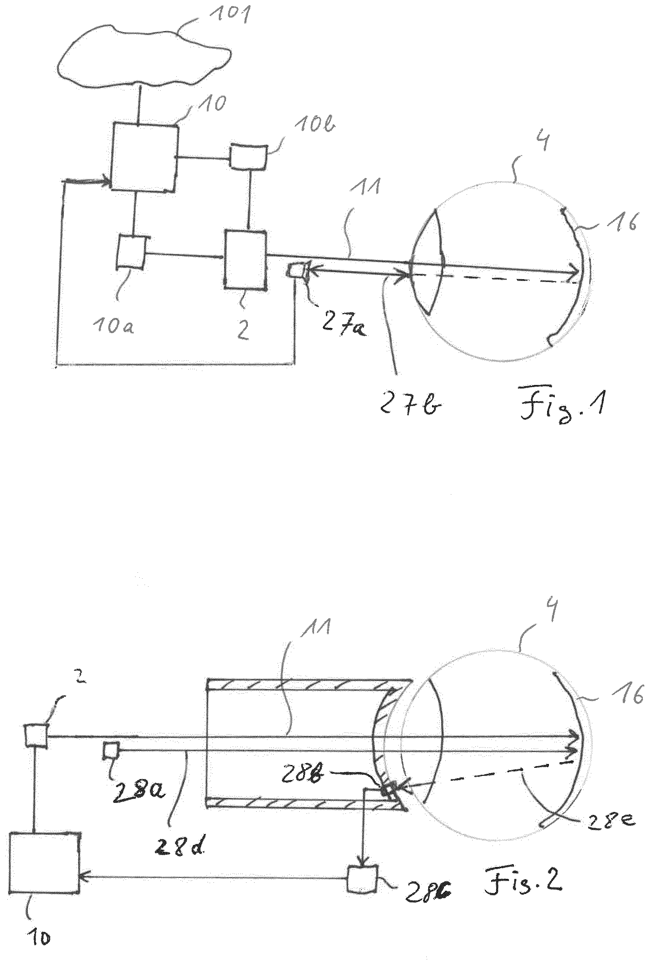

Figure 1 shows a device for tissue treatment of the retina 16 of a patient's eye 4 with a laser/treatment laser 2 which is emitting a laser beam 11 through the lens of the eye to the retina. A laser control unit 10 generally controls the direction and intensity of the laser radiation and in some cases concretely parameters like the laser power, modulation frequency, duty cycle of the laser, the laser collimation, size of the laser spot and, in some cases, also the wavelength of the laser radiation. These parameters can be controlled by the laser control unit 10 in coordination with the more specific control unit 10a. Further, the deflection of the laser beam can be controlled via a deflection control unit 10b which in some cases controls one or more deflection mirrors or other comparable deflection means. The deflection control unit 10b enables a scanning of the surface area of the retina, for example by a laser spot pattern one laser spot after the other.

In the examples shown in the figures, a laser is described but it should be noted that a laser could also be replaced in some cases by a strong light source like an LED.

The laser control unit is connected to a computer network/cloud 101 and/or to a remote server in order to exchange data, for example data of the patient who shall be treated. For example, data about the retina of the patient or other physiological data or personal data can be stored in the computer network along with a clearance for a general treatment with or without any special conditions. Further, parts of any computational efforts that could be necessary as well as any kind of software updates can be taken by a remote computer.

The device further comprises an eye tracking device 27a, 27b with a camera 27a which is directed to the eye and is arranged to observe movements of the eye. For this purpose, in some cases a light beam 27b can be used which is sent to the eye and which is reflected on the surface of the eye, wherein the reflection is observed and analysed by the camera 27a and an analysing device. Information about the eye movement is then sent to the laser control unit 10 which can adapt the deflection of the laser beam 11 accordingly in order to keep the laser beam on a target spot.

Figure 2 shows a similar treatment device as Figure 1, wherein the laser control is simplified and only a Laser 2 is shown which is controlled by a laser control unit 10. A laser beam is directed to the retina 16 of an eye 4 for treatment, for example of a macula degeneration disease.

The control of the temperature which is reached on the retina by the treatment shall remain very reliably in a certain limited temperature window. This is in many cases reached by controlling the laser power as well as other parameters of the laser. The potential absorption of the laser light by the retina can be better assessed if the retina is further analysed by taking a picture with a camera or scanner. This method is described more in detail below.

Another method of keeping the temperature limits can be implemented by measuring the temperature of the retina at or very close to the current laser spot on the retina and controlling the laser power or other laser parameters based on this information. With the temperature measurement, a closed loop control can be established. This setup is shown in Figure 2 including the laser control unit 10.

The device for this purpose comprises a tissue temperature control mechanism with a tissue temperature measurement device 28a, 28b, 28c, which sends measurement values of the retina tissue to the laser control unit 10. This way, a closed loop control can be established which makes sure that the target temperatures can be reached and kept. The temperature measurement device is based on the "Grüneisen" formula and measures the absorption of short pulses of radiation sent to the retina by an excitation laser 28a by response signals in the form of pressure waves 28e which are generated by the sudden temperature rise on the retina which is created by pulses of a the radiation beam 28d of the excitation laser. The pressure waves are captured by a pressure wave transducer 28b. The signals generated by the pressure wave transducer 28b are transformed into temperature values by a transforming unit 28c.

The excitation laser 28a can be a laser which is separate from the treatment laser, but also, in some implementations, the treatment laser itself can be used as an excitation laser for the temperature measurement.

The temperature measurement device can be used to establish a closed loop with the laser control 10 and reliably limit the temperature at a current laser spot to the target temperature. For this purpose, the laser control unit 10 can control based on the measured temperature the laser power, the duty cycle, the laser spot diameter or even the wavelength of the treatment laser.

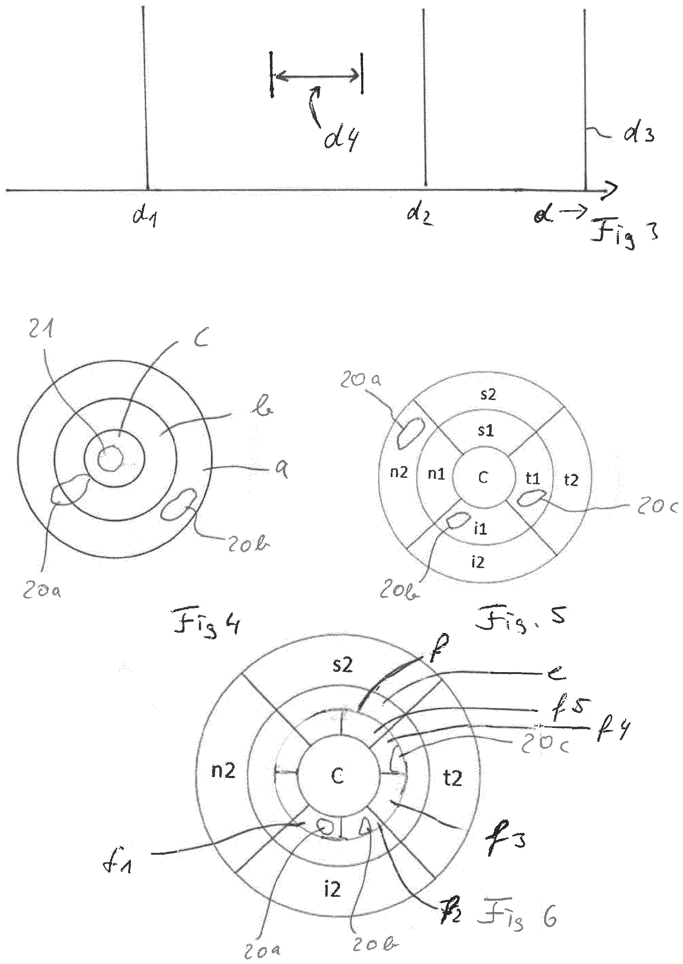

Figure 3 shows a diagram with different ranges of laser energy density d or radiation intensity on the horizontal axis.

The first vertical line at the value d1 is representing the official limit for eye security which is defined by IEC standard IEC 60825-1 at a value of 31 W/cm2. Laser power densities below this threshold are officially applicable without any risk.

The second vertical line represents d2, which is a power density well above 350W/cm2. Above this value, laser treatment may cause light damages which can be proven with sensitive diagnostic methods, but in many cases are not immediately perceivable by the patient himself.

The third vertical line represents the value d3. Laser treatments with a power density above this value cause immediate damage to the retina, which is perceivable with standard methods.

The range d4 of power densities which is claimed according to the invention is located between d1 and d2. In this range, no damage is caused at the tissue by the laser radiation, but useful physiological processes in the retina tissue are initiated, enhanced, accelerated, or at least influenced in a positive way.

Figures 4 to 6 are showing different compositions of area sections on the retina of an eye. Figure 4 simply shows two concentric circular rings a and b which are surrounding a central circular area c. The rings a and b can be area sections of the retina, whereas the area c is not covered by the area sections and shall not be irradiated because the fovea 21 is located in this central area. Two treatment area regions 20a, 20b are shown, wherein the treatment area region 20b is located in the area section a and the treatment area region 20a partially extends to both area sections a and b.

Figure 5 shows the standard ETDRS grid with an outer circle, which has four area segments i2, n2, s2, t2 forming ring segments and an inner circle with the four area sections i1, n1, s1, t1 also forming ring segments. Figure 5 further shows three treatment area regions 20a, 20b, and 20c, wherein the treatment area region 20a is located in the area section n2, the treatment area region 20b is located in the area section i1 and the treatment area region 20c is located in the area section t1.

Figure 6 shows a different potential composition of area sections, wherein an outer circular ring similarly to the composition shown in Figure 5 is divided into segments forming area sections i2, n2, s2, t2, while the inner ring is subdivided into two smaller concentric rings e and f. The outer ring e is divided into 4 area segments similar to the segments of the segments i2, n2, s2, t2, while the inner ring f is equally divided into eight ring segments each of them forming an area segment.

By this finer subdivision, the area segments, which are closer to the fovea are smaller and thus, in this region close to the fovea, which is very sensitive to potential damages, the treatment area regions can be located by assignment to area sections more accurately or more in detail.

In the example of Figure 6, the treatment area regions 20a, 20b and 20c are assigned to the area segments f1, f2 and f4.

According to the invention, a rule for irradiation of the treatment area regions may state that the treatment area regions are treated starting at f1 proceeding counter clockwise. A potential rule may also state that all those area sections containing treatment area regions will be irradiated completely, also in a counter clockwise direction or in a clockwise direction. A rule can also state in the case that between two area sections which will be irradiated (in the example of Figure 6 the area sections f2 and f4) only one area section is located which has no treatment area region (in the example of Figure 6 the area section f3) this area section will not be left out and will be irradiated anyway. However, if between two area sections which carry treatment area regions, there are at least two area sections which do not carry treatment area regions, in other words, which are not planned to be irradiated, these area sections will be skipped.

By this rule, a quick proceeding is achieved and only in single cases additional area sections will be irradiated.

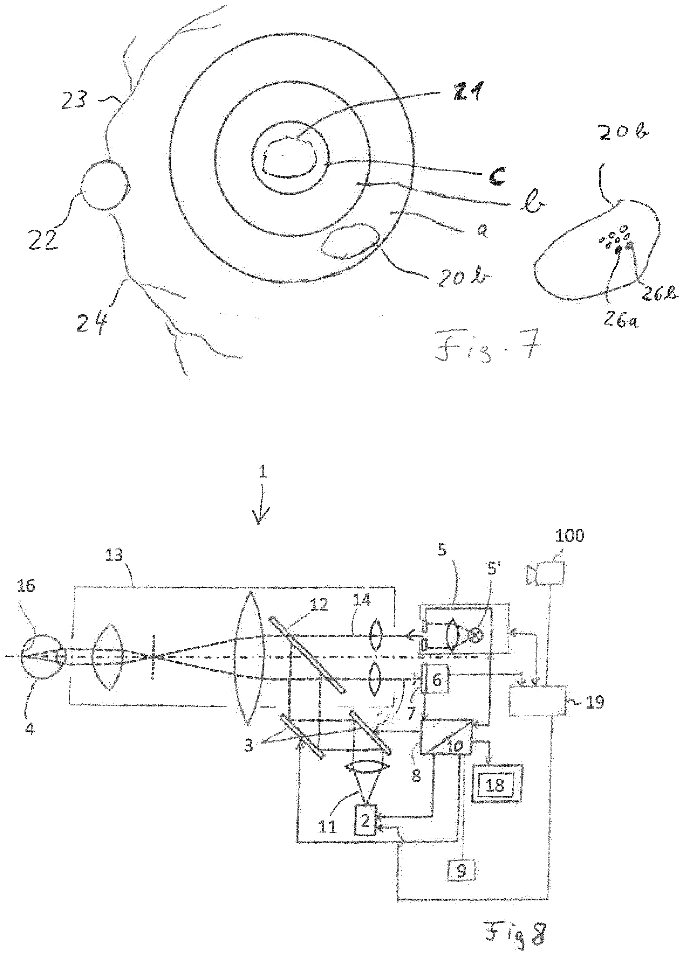

Figure 7 is showing the general setup of the area sections covering a part of the retina.

On the left side of the Figure, the optical nerve head 22 is shown at the position where it exits into the retina. From the same point, the major/primary arteries, called arcades 23, 24 are starting which support the retina with blood. The area of the retina which is covered by the area sections a, b in Figure 7 is defined by the fovea 21, which is located in the central circular area c, and by the arcades 23, 24 and the optical nerve head 22, which are located outside the outer ring a and thus, outside of the area sections.

On the right side of figure 7, it is shown as an example, how a treatment area region 20b is covered by several laser spots 26a, 26b which form a regular pattern. Patterns can be defined in advance, for example covering each of the area sections, because their shape is known and independent of any individual retina.

Figure 8 shows a device in the form of an ophthalmoscope or a device combining functions of an ophthalmoscope with functions of laser treatment of the retina 16 of a human eye 4.

The device in addition to the laser or light treatment part of the device, which is necessary for a general and potentially standardized treatment, has an illumination device 5 with a radiation source 5', which is arranged to direct an illumination beam 14 onto the eye 4 and the retina 16 of a patient. This allows the retina 16 to be suitably illuminated for capturing an image or a camera image under standardized and reproducible conditions. This allows in some cases of laser treatment to modify standard treatment parameters for a patient according to an individual measurement within certain boundaries.

The illumination may, for example, be equipped with a light emitting diode or an infrared diode as a light source, or with a light source of another type that provides a defined wavelength spectrum. The light source may also be, for example, a UV light source.

The ophthalmoscope also has a camera 6 in which a sensor 7 is provided. The sensor may be, for example, a CCD or CMOS sensor. Instead of the camera 6, any other type of device may be provided, for example, a scanning device suitable for detecting radiation reflected or scattered from the retina.

The objective in operating the illumination device 5 and the camera 6 or an equivalent device is to obtain, under defined illumination conditions, the most accurate spatially resolved measurement data possible from the retina 16 by detecting a recording of backscattered radiation, and thus to be able to detect or determine the characteristics of target areas to be treated.

The illumination beam 14 and the reflected radiation 15 are suitably collimated and focused by a suitable optical system 13 with mirrors and lenses in a well- known manner. The optical system 13 also has a beam splitter 12, which makes it possible to direct a laser beam from the treatment laser 2 onto the retina 16 using the same optical axis as the illumination beam. Alternatively, the laser beam can be coupled without a beam splitter, for example by guiding it slightly laterally offset with respect to the illumination light. A control unit 8 can be provided for controlling the laser 2, wherein the control unit 8 on the one hand controls, for example triggers, the illumination device 5, and on the other hand acquires a camera image from the camera 6 and controls the laser 2 via a laser control unit 10. The laser control unit 10 can control the laser intensity of the laser 2 as well as deflection mirrors 3, which direct the beam path of the treatment beam 11 and thus enable the targeted treatment of individual treatment area regions 20a, 20b, 20c on the retina 16.

For improved control of the laser 2, a processing device 19 is provided which permits precise processing of recordings/camera images of the camera 6 and links these to the known and defined parameters of the illumination of the retina 16.

The ophthalmoscope further comprises a sensor 100, for example in the form of a camera, which allows the measurement of the pigmentation colour and pigmentation intensity of the skin, the hair and/or the iris of the patient. An input device may also be provided to allow such measured parameters to be introduced into the system. In any case, these parameters are sent to the processing device 19, where they are taken into account in determining the values or correction values for irradiation parameters.

By illuminating the retina with known illumination parameters and linking them to the image of the retina, it is possible to assign, if necessary, correction factors for the intensity of the laser treatment for each treatment area region 20a, 20b, 20c on the retina 16 by the processing device 19 to the treatment area regions or area sections in an objectified manner. The intensity of the laser beam is therein given by the energy of the laser, the size of the laser spot on the retina and the number, repetition rate and duration of the pulse or pulses and the length of the pauses between the pulses (duty cycle of the laser).

In many areas of ophthalmology, different energy sources, particularly lasers, are used for diagnostics and treatment. As a rule, the entire beamed-in energy is absorbed by the biological tissue and converted to heat, the resulting temperature increase achieving the desired treatment effect. For example, during laser photocoagulation, the retina of the eye is thermally coagulated in a targeted manner. In the case of the conventional irradiations with irradiation times about 100 ms, temperatures of above 60° C occur. Also, in the case of transpupillary thermotherapy (TTT), temperature increases are utilized for achieving a vascular occlusion. In the case of photodynamic therapy (PDT), a previously injected dye is activated by laser irradiation of a therapy laser on the ocular fundus. The active ingredient develops its effect only on those cells to which it is bound. In this case also, almost the entire beamed-in energy is absorbed in the dye and in the retina and is converted to heat. During the respective irradiation time (pulse duration) with relatively long treatment and radiation pulses in the order of from μs to several hundred seconds, a temperature increase of the treated biological tissue, particularly of the fundus of the eye, which results in unintended damage to regions of the retina, should in any case be avoided. A non-invasive real-time temperature determination during ophthalmologic laser treatment has been desired for a long time. As a solution, a technology has been developed, which has already been shortly described with reference to Figure 2 and which allows for monitoring the tissue temperature during the thermal treatment of biological tissue, in particular during ophthalmologic treatments, by means of optoacoustic techniques. In more detail, it is known to determine the tissue temperature of biological tissue caused by a laser treatment with optoacoustic techniques by pulsed laser irradiation. Temperatures are measured at the ocular fundus for example during the selective micro photocoagulation or during any other treatment of diseases of the retina which uses an irradiation source like, for example, a therapy laser. A special laser pulse which is intended for temperature measurement generates a thermal tissue expansion or, at the end of the pulse, a thermal tissue contraction, wherein the expansion as well as the contraction generate pressure waves and wherein the amplitude or other characteristics of the pressure waves can be used for calibrating the pressure wave characteristics, e.g., the amplitude, to the temperature or to a more complex control value with a defined relation to the temperature and to an absorption characteristic of the ocular fundus. From the change of subsequent pressure wave characteristics after subsequent treatment pulses, the temperature increase or decrease and, in addition, the respective absolute temperatures can be determined by means of the relation between the temperature of the tissue and the pressure wave characteristics, in particular the pressure wave amplitude, which is defined by the Grüneisen coefficient. It is an aspect of the current invention to provide a simplified method and a device for the non-invasive determination of the control value mentioned above or of the temperature on treated biological tissue, which can also be an implementation of a control value. The control value or the measured temperature may then, as already explained above, serve for controlling the power of the therapy laser. The control value may for example be the current temperature of the treated tissue of the eye, which can be determined based on the measurement of the strength of the opto-acoustic pulses or the detected pressure waves. For example, the strength of the pressure waves, their amplitude, average amplitude or accumulated energy may be measured or determined.

The measured strength of the pressure waves depends on the tissue temperature, in which the pressure waves are generated, the absorption of the dedicated laser signals on their way to the tissue and the intensity of absorption of the laser signals in the tissue (hence, for example: the pigmentation strength). In the range of parameters, where the treatment generally takes place, a variation of the strength of the detected pressure waves dP is determined by the following equation:

$$dP=\mu\,\Gamma\,dH,$$

wherein dP is the Variation of the strength of the pressure waves, $\mu$ is the absorption strength of laser light in the tissue, $\Gamma$ is the Grüneisen coefficient and dH is the accumulated laser beam energy in the tissue (heat dissipation in the tissue has been neglected for short time ranges). Because both parameters, the tissue temperature and the absorption strength of the tissue, point into the same direction (high temperature and high absorption strength require less power of the therapy laser), the measured strength of the pressure waves is an appropriate control value for controlling the power of the therapy laser. Therein, it is one option to use the control value as such for the control of the therapy laser, but it is another option to first determine the current temperature of the tissue, for example based on a calibration, and after that to control the therapy laser based on the determined temperature value.

**Claims**

1. Device for tissue treatment of the retina (16) of a patient's eye (4) by means of a controllable light source(2), in particular a laser, the light beam (11) of which can be directed onto different treatment area regions (20a, 20b, 20c) of the retina, **characterized in that** the device is set up to heat the retina spot (26a, 26b) by spot, within one or more treatment area regions, successively by irradiation with the light source for a controllable period of time to a temperature between 48 and 58 degrees Celsius, in particular between 50 and 55 degrees Celsius.

2. Device for tissue treatment of the retina (16) of a patient's eye (4) by means of a controllable light source (2), in particular a laser, the light beam (11) of which can be directed onto different treatment area regions (20a, 20b, 20c) of the retina, **characterized in that** the device is set up to heat up one or more treatment area regions in a manner that does not lead to a damaging or coagulation of tissue of the retina but leads to the release of a cascade of biochemical reactions, in particular leading to an improved metabolism of the RPE, further in particular leading to the release of heat shock proteins in or near the retina.

3. Device according to claim 1 or 2, **characterized in that** the device is arranged to heat the retina (16) spot (26a, 26b) by spot successively by irradiation with the light source (2) for a period of less than 500 msec, in particular less than 200 msec per spot, to a temperature between 48 and 58 degrees Celsius, in particular between 50 and 55 degrees Celsius.

4. Device for tissue treatment of the retina (16) of a patient's eye (4) by means of a controllable light source (2), in particular a laser the light beam (11) of which can be directed onto different treatment area regions (20a, 20b, 20c) of the retina, **characterized in that** the device is set up to irradiate the retina spot (26a, 26b) by spot, within one or more treatment area regions, successively by irradiation with the light source for a period of less than 500 msec, in particular less than 200 msec per spot, with a power density of between 100 and 350 W/cm$^2$, in particular between 120 and 300 W/cm$^2$.

5. Device according to claim 1, 2, 3 or 4, **characterized in that** the device is arranged to assign the treatment area regions (20a, 20b, 20c) to be treated with the light source to predefined area sections (n1, s1, t1, i1, n2, s2, t2, i2) and to irradiate them according to a sequence determined by the assignment of the treatment area regions to the area sections and/or that at least one group of area sections (n1, s1, t1, i1, n2, s2, t2, i2) are arranged next to one another in the circumferential direction around the fovea and/or **in that** the area sections together cover an area of the retina surrounding the fovea (21), the extent of which is in particular such that it does not cover the exit site of the optic nerve (22) and in particular the arcades (23, 24).

6. Device according to one of the claim 5, **characterized in that** the light source control is set up to irradiate a group of area sections (n1, s1, t1, i1, n2, s2, t2, i2), in particular all area sections individually one after the other, either not at all or to cover 100% of the individual area section.

7. Device according to one of claims 5 or 6, **characterized in that** at least one group of area sections (n1, s1, t1, i1, n2, s2, t2, i2) is arranged next to one another in the circumferential direction around the fovea.

8. Device according to any one of claims 5 to 7, **characterized in that** the area sections (n1, s1, t1, i1, n2, s2, t2, i2) are distributed around the fovea (21), in particular in the form of segments of at least one circular ring (a, b) concentrically surrounding the fovea or of an elliptical ring surrounding the fovea, or **in that** the area sections are arranged on one or more segments of a ring, in particular circular ring, concentrically surrounding the fovea, wherein the area sections are arranged rotationally symmetrically with an n-numbered rotational symmetry, wherein n is in particular between 1 and 9 and is further in particular 2, 4 or 6.

9. Device according to one of claims 1 to 8, **characterized in that** the device is set up to record areas of the retina (16) with an imaging method, to select treatment area regions (20a, 20b, 20c) on the basis of an image of the retina, to assign irradiation parameters to these and to irradiate these using the irradiation parameters.

10. Device according to one of the claims 5 to 9, **characterized by** a retina detection device (6, 7) which records an image of the predefined area sections (n1, s1, t1, i1, n2, s2, t2, i2) of the retina by means of an imaging process, in particular by means of a camera or an optical scanner, and assigns irradiation parameters associated in each case with the area sections.

11. Device according to one of claims 1 to 10, **characterized by** an eye-tracking device (25) which compensates for movements of the eye (4) during the irradiation of the treatment areas regions (20a, 20b, 20c) and/or by a temperature measurement device comprising a thermo-optic spectrometer (28a, 28b, 28c) which sends laser pulses to the retina and registers pressure waves being emitted by the retina as a response signal.

12. Method for tissue treatment of the retina of a patient's eye (4) by means of a controllable light source, in particular a laser (2), wherein the light beam (11) can be directed onto different treatment area regions (20a, 20b, 20c) of the retina (16), **characterized in that** within one or more treatment area regions the retina is heated spot (26a, 26b) by spot one after the other by irradiation with the light source for a controllable period of time to a temperature between 48 and 58, in particular between 50 and 55 degrees Celsius and/or that the device is set up to heat up treatment area regions in a manner that does not lead to a damaging or coagulation of tissue of the retina but leads to the release of a cascade of biochemical reactions, in particular leading to an improved metabolism of the RPE, further in particular leading to the release of heat shock proteins in or near the retina.

13. Method for tissue treatment of the retina of an eye of a patient by means of a controllable light source, in particular a laser (2), wherein the light beam (11) can be directed onto different treatment area regions (20a, 20, 20c) of the retina (16), **characterized in that** within one or more treatment area regions the retina is irradiated spot (26a, 26b) by spot in succession by irradiation with the light source for a period of less than 500 msec, in particular less than 200 msec per spot, with a power density between 100 and 350 W/cm$^2$, in particular between 120 and 300 W/cm$^2$.

14. Method according to claim 12 or 13, **characterized in that** the treatment area regions (20a, 20b, 20c) to be treated with the light source are assigned to predefined area sections (n1, s1, t1, i1, n2, s2, t2, i2) and are irradiated according to a sequence determined by the assignment of the treatment area regions to the area sections.

15. Method according to one of claims 12 to 14, **characterized in that** first an image of predefined area sections (n1, s1, t1, i1, n2, s2, t2, i2) of the retina is recorded by means of a retina recording device (6, 7) by an imaging method and irradiation parameters associated with the entire retina or with the area sections or treatment area regions (20a, 20b, 20c) determined within these are assigned.

Fig.1

Fig.2

Fig 3

Fig 4

Fig. 5

Fig 6

Fig. 7

Fig 8

Europäisches
Patentamt

European
Patent Office

Office européen
des brevets

# EUROPEAN SEARCH REPORT

**Application Number**

EP 23 19 8985

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2013/116672 A1 (YEE KINGMAN [US]) 9 May 2013 (2013-05-09) * paragraphs [0004], [0026], [0027], [0032], [0033], [0042], [0049], [0052], [0058], [0062] * | 1-3,5-15 | INV. A61F9/008 |
| X | US 2006/100677 A1 (BLUMENKRANZ MARK S [US] ET AL) 11 May 2006 (2006-05-11) * figure 6b * * paragraphs [0001], [0002], [0016], [0017], [0024], [0036] * | 1-8, 12-15 | |
| X | WO 2017/192168 A1 (OJAI RETINAL TECH LLC [US]) 9 November 2017 (2017-11-09) * paragraphs [0001], [0071], [0087], [0106], [0107], [0117], [0121] * | 1-7,9-15 | |

TECHNICAL FIELDS
SEARCHED (IPC)

A61F

The present search report has been drawn up for all claims

| Place of search | Date of completion of the search | Examiner |
|---|---|---|
| Munich | 5 March 2024 | Jansen, Birte |

EPO FORM 1503 03.82 (P04C01)

**EP 4 527 363 A1**

## ANNEX TO THE EUROPEAN SEARCH REPORT
## ON EUROPEAN PATENT APPLICATION NO.

EP 23 19 8985

05-03-2024

| Patent document cited in search report | | | Publication date | Patent family member(s) | | Publication date |
|---|---|---|---|---|---|---|
| US 2013116672 | A1 | | 09-05-2013 | CN | 103997948 A | 20-08-2014 |
| | | | | EP | 2768378 A1 | 27-08-2014 |
| | | | | JP | 5882486 B2 | 09-03-2016 |
| | | | | JP | 2014534011 A | 18-12-2014 |
| | | | | US | 2013110092 A1 | 02-05-2013 |
| | | | | US | 2013110093 A1 | 02-05-2013 |
| | | | | US | 2013110206 A1 | 02-05-2013 |
| | | | | US | 2013116672 A1 | 09-05-2013 |
| | | | | US | 2016128871 A1 | 12-05-2016 |
| | | | | US | 2016228295 A1 | 11-08-2016 |
| | | | | US | 2017304119 A1 | 26-10-2017 |
| | | | | WO | 2013059564 A1 | 25-04-2013 |
| US 2006100677 | A1 | | 11-05-2006 | AU | 2004311625 A1 | 21-07-2005 |
| | | | | DK | 1701651 T3 | 19-01-2015 |
| | | | | EP | 1701651 A2 | 20-09-2006 |
| | | | | ES | 2526402 T3 | 12-01-2015 |
| | | | | FI | 9630 U1 | 19-04-2012 |
| | | | | IL | 176393 A | 30-12-2010 |
| | | | | JP | 4377405 B2 | 02-12-2009 |
| | | | | JP | 2006524515 A | 02-11-2006 |
| | | | | US | 2006100677 A1 | 11-05-2006 |
| | | | | US | 2010249760 A1 | 30-09-2010 |
| | | | | US | 2010256615 A1 | 07-10-2010 |
| | | | | US | 2014081248 A1 | 20-03-2014 |
| | | | | US | 2016262933 A1 | 15-09-2016 |
| | | | | WO | 2005065116 A2 | 21-07-2005 |
| WO 2017192168 | A1 | | 09-11-2017 | AU | 2016405579 A1 | 04-10-2018 |
| | | | | BR | 112018072711 A2 | 19-02-2019 |
| | | | | CA | 3018167 A1 | 09-11-2017 |
| | | | | CN | 109069294 A | 21-12-2018 |
| | | | | EP | 3451983 A1 | 13-03-2019 |
| | | | | EP | 3593771 A1 | 15-01-2020 |
| | | | | ES | 2951189 T3 | 18-10-2023 |
| | | | | ES | 2952701 T3 | 03-11-2023 |
| | | | | JP | 2019514454 A | 06-06-2019 |
| | | | | SG | 11201808069V A | 29-11-2018 |
| | | | | WO | 2017192168 A1 | 09-11-2017 |

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1875857 A1